# EUROPEAN PATENT APPLICATION

(11) **EP 2 080 478 A1**
(43) Date of publication of application: **22.07.2009**
(21) Application number: 08100529.0
(22) Date of filing: 16.01.2008
(51) Int. Cl.: A61B 8/08, A61B 17/34, A61B 1/00

(54) **Device for guiding an invasive medical instrument**

(71) Applicant: Giesen Design Consultancy BV, 4835 JL Breda (NL)
(72) Inventor: Giesen, Franciscus Jozef Hermanus, 4835 JL, Breda (NL)
(74) Representative: Grootscholten, Johannes A.M.

(57) **Abstract**

The present invention relates to a device (1) for guiding a invasive medical instrument (4), such a needle, a syringe, a biopter, or the like, relative to an imaging apparatus (3). The imaging apparatus is essentially non-invasive. The device of the invention comprises an accommodation (2) for the imaging apparatus. Also the device has a guide (5) adjacent the accommodation for guiding the medical device relative to the imaging apparatus. Thus accurate positioning of the medical instrument is enabled, while keeping the needle in the line of sight of the imaging apparatus.
In a particularly preferred embodiment, a guide surface (7) is defined, and where opposite the guide surface at least partially resilient press means (8) are provided, which in use press the medical instrument against the guide surface. Thus medical instruments having different sizes or diameters can be employed. The risk of any such medical instrument, like a needle breaking, is greatly reduced.

## Description

The present invention relates to a device for guiding an invasive medical instrument, such a needle, a syringe, a biopter, or the like, relative to an imaging apparatus, which is essentially non-invasive.

It is noted that through forming images of the interior of a patient with the aid of the imaging apparatus and viewing the formed images, an operator or physician utilising the medical instrument is able to better position the medical device in the interior of a body of a patient, since on the basis of the formed images, the operator is accurately informed of the position reached by the medical device operated by the operator. Especially in relation to organs or other sub-dermal locations accurate positioning of the medical instrument is often highly relevant or extremely important. For example, continuous visualisation of a needle or other invasive instrument, such as during anaesthesia, during ultrasound guided interventions is highly important when inserting the invasive device into tissues which may be in close proximity to vessels, pleura or nerves. Without accurate identification and more in particular localization of the needle damage to collateral structures may be caused. Thus the effectiveness of use of the invasive medical instruments is improved, when used in conjunction with an imaging instrument.

As examples of imaging instruments here are mentioned the likes of ultrasound probes, computed tomography scanners (CT Scanners), and magnetic resonance imagers (MRI). Such non-invasive imaging devices have enabled considerable improvements in the effectivity of diagnostics and accuracy of medical procedures.

For example, ultrasound probes and other imaging instruments are also being more and more used for a variety of purposes, such imaging a fetus, identifying the existence, location, and size of tumors, as well as the existence of other medical conditions. Ultrasound probes are also known to be used when inserting catheters into blood vessels and organs.

For many imaging applications, it is desirable that an invasive instrument or device is used to apply actual therapeutic treatment, the medical device comprising a needle, biopsy instrument, a biopter, a catheter, or other more or less thin invasive instrument. In view of the common invasive nature of such medical instruments, these may herein below generally be referred to as needles, although these may be used to perform different medical applications, other than to just inject or retract fluid. For instance the needles can be inserted into the body of a patient in order to remove a biopsy sample.

Thus it is normally desirable that the needle be guided to a specific position within the body of the patient. Often such a specific position is located using conventional diagnostics methods or even the imaging apparatus.

Nonetheless, operators or physician have hitherto been left to deal with this matter for themselves in that have had to manoeuvre the needle while handling the imaging device to obtain images of the position reached by the (tip of the) medical instrument.

The need to have to simultaneously handle the imaging apparatus and manoeuvre the medical instrument, both manually and individually, requires skill and is nonetheless still highly cumbersome. Often the medical instrument is lost from view on a monitor connected with the imaging apparatus for forming the images, especially with so-called IP or in-plane technique. If this happens the operator or physician has the possibility of retracting the medical instrument and start all over again, with all evident discomfort to the patient as a direct result. Also the imaging apparatus can be displaced to find the (tip of the) medical instrument again, which is something that arouses suspicion with the patient, since the operator or physician would then evidently be looking for a lost needle, at least in the perception of the patient.

It is noted that for positioning the medical instrument relative to an imaging apparatus two methods are predominantly employed. One of these is a so-called in-plane (IP) technique, mentioned directly above, in which the entire needle and the tip can be visualised, or the out-of-plane (OOP) technique. The OOP technique results in the needle being imaged on cross-section, which has the disadvantage that the needle will cross the ultrasound beam only once. The major obstacle for the in-plane IP technique is to keep the needle exact in the path of the ultrasound beam.

The present invention has for an object to obviate or at least lessen these drawbacks of the prior art, to which end a guiding device is proposed herein in accordance with the present invention, as defined in the appended independent claim.

With a device according to the present invention it has become possible to maintain the medical instrument within the imaging zone corresponding with the imaging apparatus, such as an ultrasound device. As a result of the positional relationship between the imaging apparatus and the invasive medical instrument, keeping the (chip of the) medical instrument within the line of sight of the imaging apparatus has been considerably improved.

Within the bounds of the present invention as defined in the appended independent claim, several preferred embodiments are possible, which are noted herein below as well, however without presenting such preferred embodiments as limiting the invention in any way.

For instance it is possible, that the guide comprises a slot, which is oriented in such a direction to pass the medical instrument there through for manoeuvring the medical instrument essentially in an imaging zone corresponding with the imaging apparatus. Providing a slot for manoeuvring the medical instrument has for an advantage, that manoeuvring can be restricted essentially to a plane in the centre below the imaging apparatus, thus restricting the freedom of movement of the medical instrument to said plane.

In a further preferred embodiment, the guide comprises at least one guide surface defining an area for at least a part of the medical instrument to abut there against. Rather than defining a slot, or in addition thereto, such an abutment surface provided for the medical instrument can allow for some degree a freedom when deviations from the centre plane relative to the imaging apparatus are desired. Nonetheless, if centralisation of the medical instrument is desired, abutment against the guide surface can provide the desired guidance in the desired orientation within the plane or centre plane corresponding with the imaging apparatus. However, if only a guide surface having a guide area is provided, then it may be desirable to provide an additional guide surface. This may, in combination with the first guide surface, form a slot or any other shape to keep the medical instrument in the space between the two guide surfaces. However, additionally or alternatively opposite the provided guide surface having the guidance area at least partially resilient press means are provided, which in use press the medical instrument against the guide surface. The feature, that the press means are at least partially resilient, allows for the use of different medical instruments, having different thicknesses or diameters. Thus it has become possible in this embodiment to employ a single device according to the present invention in combination with different medical instruments each having its own particular thickness. Otherwise, different devices according to the invention, each having the capacity to be employed in combination with a medical instrument having a specific thickness or diameter, would have to be provided. In an embodiment with press means, these could comprise as a nonlimiting example a leg extending over essentially the same length as the guide surface in a direction away from the accommodation. Thus it possible to manoeuver the medical instrument within a range of movement, defined by the combination of the leg and the guide surface or area. Such press means serve as a inventive solution to yet another problem, which is that of safety. An invasive medical instrument, like a needle, is rigid and breakable. If during insertion thereof through a stiff slot, e.g. enclosed on opposite sides by inflexible walls, the needle follows an oblique path relative to the angle of insertion, which determined by the stiff slot, the needle could break, causing all kinds of harm in the process. This is effectively prevented by a resilient press means which are suitable for allowing the needle to bend or be deflected, without breaking. Also or alternatively, the press means could comprise a leg extending under an angle relative to the direction of the medical instruments passing along the guide surface. With the leg inclined towards the guide surface, the function of the press means to keep the medical instrument forced against the guide surface or area is improved. Further, in such an embodiment, it is possible that the leg is fixed relative to the guide surface at a position at a larger distance from the guide surface than the angled portions of the leg. This could provide the desired resilience to the leg and thus form efficient press means, which could be embodied in a unitary form, together with the guide surface and preferably also with the accommodation for the imaging apparatus. Especially, when the angled portions of the leg form a cantilever, this function could be optimized.

Herein below a preferred embodiment of a device according to the present invention is described in conjunction with the accompanying drawings, where it is noted that the shown and described embodiment is not limiting upon the invention as defined in the claims, and where the same or similar elements, component and features are designated with the same reference numbers. In the drawings:
Figure 1 shows a perspective view of the device according the present invention in use;
Figure 2 shows a detail in figure 1 in accordance with arrow II;
Figure 3 shows an elevated front view in accordance with arrow III in figure 1;
Figure 4 shows the device of figure 1 in isolation; and
Figure 5 shows a view from below on the device in accordance with figure 4 along arrow V in figure 4.

In the figures a device 1 according to the present invention is shown in use, comprising a holder 2 forming an accommodation for an imaging apparatus, such as an ultrasound probe 3. The ultrasound probe 3 is used in conjunction with a schematically shown medical instrument 4, such as a syringe, a needle, a cavitor, a biopter, etc. For optimal positioning of the medical instrument 4 relative to the field of view created by the ultrasound probe 3, the holder 2 is connected with a guide 5, and preferably forms a unitary structure therewith. The guide 5 defines a slot 6 for introducing the medical instrument 4 there through. More in particular, the guide 5 comprises a guide surface 7, against which a portion of the length of the medical instrument 4 can abut. By keeping the portion of the medical instrument 4 against the guide surface 7, alignment of the medical instrument 4 in relation to the field of view of the ultrasound probe 3 is achieved.

In specific embodiments the slot 6 can be defined by two opposite guide surfaces 7.

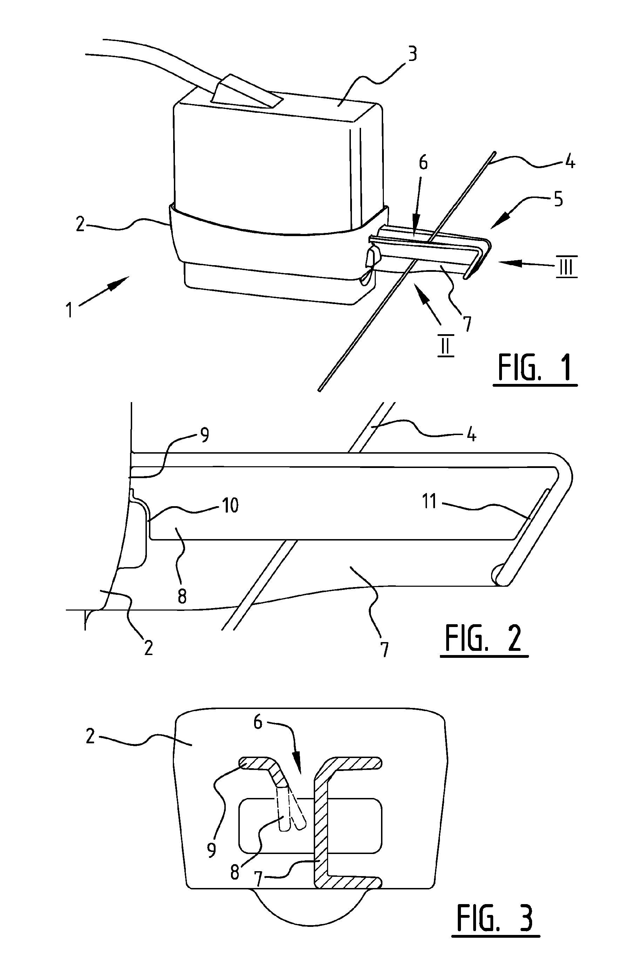

However, as shown in figures 2 and 3, in the shown and described embodiment, a cantilever plate 8 is provided opposite the guide surface 7, which plate 8 is inclined, in a relaxed state, toward the guide surface 7. A medical instrument 4, introduced in the slot 6, is thereby pressed against the guide surface 7. The cantilever plate 8, which is resiliently suspended from an attachment 9, between the cantilever plate 8 and the holder 2, thus forms press means in a sense that the medical instrument 4 introduced into and through the slot 6 is pressed over a specific length against the guide surface 7. Other resilient or spring based press means are equally well suited to be embodied in a device according to the invention.

As shown in more detail in figure 2, the cantilever plate 8 is defined by cuts 10, whereby the cantilever plate 8 is provided with a desired degree of resilience to allow for medical instruments of different sizes to be introduced there through, and to allow for deflection of plate 8 when the instrument is bent or forced sideways. The attachment 9 is, in the embodiment of figure 3, farthest away from the guide surface 7.

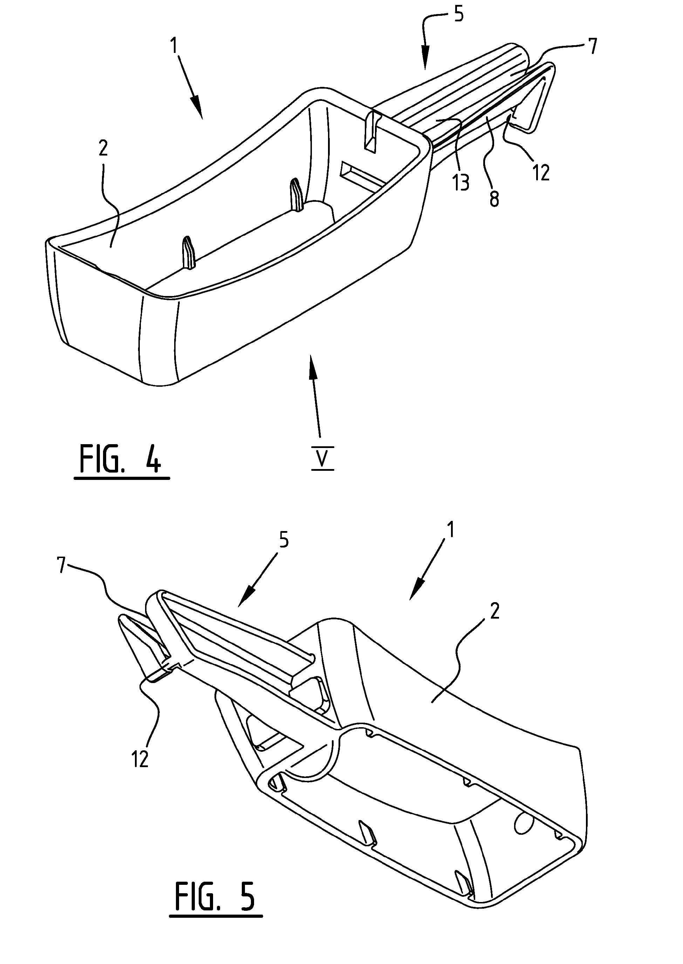

In figure 4, the device 1 of figure 1 is shown in isolation i.e. without the ultrasound probe 3 and without the medical instrument 4. At the distal end of the guide 5 relative to the holder 2, the leg 13 from which the cantilever plate 8 is suspended, is shown to be attached to the guide surface 7 at a connection 12. Thus, the leg 13 with the cantilever plate 8 suspended therefrom will be prevented from diverging away from the guide surface 7, when a relatively thick medical instrument 4 is introduced into the slot between the guide surface 7 and the cantilever plate 8. The connection 12 is again more clearly represented in figure 5, which also makes abundantly clear that the bottom of the holder 2 is open for the ultrasound probe to be able to emit waves and receive reflections in order to be able to form images from a received response on a monitoring device (not shown).

After having disclosed the present invention, at least a single embodiment thereof, in the description above in conjunction with the accompanying drawings, many additional and alternative embodiments will have become apparent to the skilled person. Thus, it is evident that many alternative and additional embodiments are possible within the framework of the present invention as defined in the accompanying claims, especially the independent claim, and should only then be accepted as anything other than embodiments of the present invention, if such alternative and/or additional variants depart from the letter or spirit of the appended claims. For instance, th underside of the holder 2 is described as open. In an alternative embodiment the holder 2 could comprise at the bottom thereof, when is in use directed at the patient, be provided with a preferably thin foil, or any other type of component for closing the bottom of holder 2 while still be able to allow waves (radio, ultrasound or other waves) to pass there through. With such a feature, resembling a membrane or the like, is that such a sheet or membrane can extend over the sides of the imaging apparatus and of the holder. Thus sterile conditions can be maintained and ensured.

The imaging apparatus needs to be used, in many forms and shapes of such imaging apparatuses, sterile. Normally a sheet or protective member is arranged over the imaging apparatus, by way of an adhesive or the like, on one side of such a sheet or protective member. According to the invention, such a protective sheet or member can be provided as an integral part of the device and/or form the foil, sheet or membrane mentioned directly above. In fact, the device according to the invention can itself be integrated as a unit with the imaging apparatus. In a space defined by holder 2, imaging apparatus 3 and a protective sheet or membrane a material conductive to the waves of the imaging apparatus can be introduced. Also a protective sheet or membrane can be provided all around the device according to the invention, and thus enclose the conductive material, to be opened at the upper side of the device just prior to use. Thereafter the imaging apparatus can be introduced into the holder on the opened upper side thereof and into the conductive material. The protective sheet could even be arranged on the device with or without the conductive material, to be entirely removed just prior to use, and to open also the lower side of the device of the invention just prior to use. However, the sheet on this lower side could just as well remain to close off the lower side of the apparatus in use also. Further, two opposite guide surfaces can also be used rather than employing press means, for instance in the form of the resilient cantilever plate. The cantilever plate 8 could be made from a material, which is in itself more resilient than other parts of the device, to optimally enclose inserted medical instrument 4 of different diameters optimally between the cantilever plate and the guide surface 7. In such an embodiment with another material, or in embodiment with sufficiently resilient material, the cuts 10, 11 can be obsolete and omitted. Moreover, any configuration pressingly engaging the medical instrument to position the instrument in a desired orientation in at least one direction relative to the imaging apparatus can be considered as an embodiment of the invention with press means, even a simple spring based configuration. A similar guide as the one shown in the figures, can also be provided along the longer side of the holder 2 forming an accommodation for an imaging apparatus, or can extend therefrom.

## Claims

1. Device for guiding a invasive medical instrument, such a needle, a syringe, a biopter, or the like, relative to an imaging apparatus, which is essentially non-invasive, comprising:
- an accommodation for the imaging apparatus; and
- a guide adjacent the accommodation for guiding the medical device relative to the imaging apparatus.

2. Device as claimed in claim 1, wherein the guide comprises a slot which is oriented in such a direction to pass the medical instrument therethrough for manoeuvring the medical instrument essentially in an imaging zone corresponding with the imaging apparatus.

3. Device as claimed in claim 1 or 2, wherein the guide comprises at least one guide surface defining an area for at least a part of the medical instrument to abut there against.

4. Device as claimed in claim 3, wherein opposite the guide surface an additional guide surface is provided.

5. Device as claimed in claim 3, wherein opposite the guide surface at least partially resilient press means are provided, which in use press the medical instrument against the guide surface.

6. Device as claimed in claim 5, wherein the press means comprise a leg extending over essentially the same length as the guide surface in a direction away from the accommodation.

7. Device as claimed in claim 5 or 6, wherein the press means comprise a leg extending under an angle relative to the direction of a medical instrument passing along the guide surface.

8. Device as claimed in claim 7, wherein the leg is fixed relative to the guide surface at a position at a larger distance from the guide surface than the angled portions of the leg.

9. Device as claimed in claim 7 or 8, wherein the angled portions of the leg form a cantilever.

10. Device as claimed in any one of the preceding claims, wherein the bottom of the holder is closed with a member made from a material to allow waves from the imaging apparatus to pass there through.
